(19) Europäisches Patentamt
European Patent Office
Office européen des brevets

(11) Veröffentlichungsnummer: **0 210 499 B1**

(12) **EUROPÄISCHE PATENTSCHRIFT**

(45) Veröffentlichungstag der Patentschrift: **09.10.91**

(51) Int. Cl.5: **C12N 11/04**

(21) Anmeldenummer: **86109483.7**

(22) Anmeldetag: **11.07.86**

(54) **Verfahren zur Immobilisierung von Enzymen.**

(30) Priorität: **27.07.85 DE 3527014**

(43) Veröffentlichungstag der Anmeldung:
**04.02.87 Patentblatt 87/06**

(45) Bekanntmachung des Hinweises auf die Patenterteilung:
**09.10.91 Patentblatt 91/41**

(84) Benannte Vertragsstaaten:
**AT BE CH DE FR GB IT LI NL SE**

(56) Entgegenhaltungen:
**EP-A- 0 052 829**
**DE-A- 2 835 874**
**US-A- 4 334 027**

**DIE ANGEWANDTE MAKROMOLEKULARE CHEMIE, Band 91, November 1980, Seiten 161-178, Heidelberg, DE; L. D'ANGIURO et al.: "Enzyme immobilization on polyglycidylmethacrylate graft-copolymer of different polysaccharides"**

(73) Patentinhaber: **BASF Aktiengesellschaft**
**Carl-Bosch-Strasse 38**
**W-6700 Ludwigshafen(DE)**

(72) Erfinder: **Marcinowski, Stefan, Dr.**
**Weimarer Strasse 82**
**W-6700 Ludwigshafen(DE)**
Erfinder: **Sanner, Axel, Dr.**
**Lorscher Ring 2c**
**W-6710 Frankenthal(DE)**
Erfinder: **Tschang, Chung-Ji, Dr.**
**Hinterbergstrasse 31**
**W-6702 Bad Dürkheim(DE)**
Erfinder: **Ladner, Wolfgang, Dr.**
**Adolf-Diesterweg-Strasse 141**
**W-6700 Ludwigshafen(DE)**
Erfinder: **Greif, Norbert, Dr.**
**Im Woogtal 3**
**W-6719 Bobenheim(DE)**

EP 0 210 499 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Immobilisierung von Enzymen.

Immobilisierte Enzyme und deren Anwendung sind bereits bekannt (vgl. Methods in Enzymology Vol. 44: Immobilized Enzymes, Academic Press 1976 sowie Immobilized Enzymes, Halsted Presss 1978).

Um die Immobilisierung von Enzymen möglichst einfach und ohne großen Aufwand zu erreichen, wurde bereits versucht, diese in Gele einzubetten. Ein Beispiel hierfür ist die Herstellung eines enzymhaltigen wäßrigen Gels unter Verwendung von Acrylamid und Methylen-N,N'-bisacrylamid (Acta Chem. Scan. 20, 2807, (1966)). In einem solchen Gel ist das immobilisierte Enzym jedoch nur mechanisch eingeschlossen, ohne kovalent gebunden zu sein. Dies hat zur Folge, daß beim Gebrauch des Gels das Enzym in erheblichem Maße extrahiert wird. Um dies zu vermeiden, kann das Enzym mit solchen vinylgruppenhaltigen Monomeren umgesetzt werden, die beispielsweise mit Amino- oder Hydroxylgruppen des Enzyms zu reagieren vermögen (Methods in Enzymology, loc. cit.). Nach einer solchen Behandlung verhält sich das Enzym wie ein vinylgruppenhaltiges Monomeres und wird bei der Polymerisation kovalent in das entstehende Gel eingebaut. Bei diesem Verfahren ist jedoch nachteilig, daß für die Herstellung des unlöslichen Enzympräparates ein zusätzlicher Verfahrensschritt benötigt wird und daß die geeigneten Monomeren meist schwer zugänglich und der enzymatischen Aktivität abträglich sind. Ein Verfahren, welches alle denkbaren Nachteile möglichst vollständig zu vermeiden sucht, ist in der DE-OS 28 35 874 beschrieben. Hiernach wird eine enzymatisch aktive Biomasse erst mit einem polyfunktionellen Epoxi-Prepolymer, dann mit einem mehrfunktionellen Amin gemischt und schließlich über mehrere Stufen unter Zuhilfenahme eines Polyelektrolyten in ein makroporöses, perlförmiges Präparat übergeführt. Dieses Verfahren ist jedoch sehr kompliziert und erfordert bei seiner Realisierung im technischen Maßstab einen außerordentlich großen apparativen Aufwand.

Der vorliegenden Erfindung liegt die Aufgabe zugrunde, ein möglichst einfaches Verfahren der Immobilisierung zu schaffen, das die Nachteile der oben angeführten Verfahren vermeidet.

Gegenstand der Erfindung ist ein Verfahren zur Immobilisierung von Enzymen oder Mikroorganismen, dadurch gekennzeichnet, daß man folgende Bestandteile in wäßriger Lösung miteinander mischt:

a) ein Enzym oder einen dispergierten Mikroorganismus

b) einen wasserlöslichen Glycidylether, hergestellt durch Anlagerung von 1 bis 30 Äquivalenten eines Epoxids der Formel I

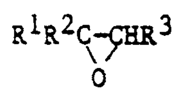

I,

worin die Reste $R^1$, $R^2$ und $R^3$ Wasserstoffatome oder Methyl- oder Ethylgruppen darstellen, an 1 Äquivalent (bezogen auf OH-Gruppen) eines polyfunktionellen Alkohols mit 2 bis 6 Kohlenstoffatomen oder eines Mono- oder Disaccharids, Umsetzung des so erhaltenen Anlagerungsproduktes mit Epichlorhydrin und anschließende Cyclisierung zum Epoxid, und

c) ein wasserlösliches Amin mit mindestens zwei N-H-Gruppen, gegebenenfalls in partiell neutralisierter Form,

und die so erhaltene Mischung gelieren läßt.

Die zur Immobilisierung verwendeten Enzyme müssen nicht in reiner Form vorliegen. Es können auch Lösungen, die Zellbruchstücke enthalten, eingesetzt werden. Es kommen jedoch nur solche Enzyme in Frage, die unter den pH-Bedingungen der Gelierung (pH 7,5-11) stabil sind. Geeignete Enzyme oder enzymhaltige Produkte sind: Glucoseisomerase, alkalische Phosphatase, Alkoholdehydrogenase, Elastase, D-Hydantoinase, Leucinaminopeptidase, Phosphodiesterase, Proteasen, Asparaginase, Esterase, beta-Galactose-Dehydrogenase, Glycerokinase, Lipasen, D-Aminosäureoxidase, Arginase, Nuclease und Urikase.

Der wasserlösliche Glycidylether, den man aus dem Epoxid, dem polyfunktionellen Alkohol oder dem Mono- oder Disaccharid und Epichlorhydrin erhält, besitzt die Formel II

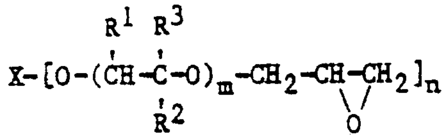 II,

worin m eine Zahl von 1 bis 30, n eine ganze Zahl von 2 bis 10, X der Rest des polyfunktionellen Alkohols oder des Mono- oder Disaccharids sind und $R^1$, $R^2$, $R^3$ die oben genannte Bedeutung haben. n entspricht in der Regel der Zahl der OH-Gruppen in dem polyfunktionellen Alkohol oder in dem Mono- oder Disaccharid.

Die Reste $R^1$ bis $R^3$ sind bevorzugt Wasserstoff. In der Formel II müssen die Substituenten $R^1$ bis $R^3$ nicht an allen $-R^1CH-CR^2R^3-O-$Einheiten identisch sein. Verbindungen mit verschiedenen Kettengliedern erhält man z.B. durch Kondensation verschiedener Epoxide.

Von der Gesamtzahl der Reste $R^1$, $R^2$ und $R^3$ sollten die Hälfte, vorzugsweise zwei Drittel Wasserstoffatome sein, damit die Produkte wasserlöslich sind. Bevorzugte Epoxide sind Propylenoxid, Butylenoxid und insbesondere Ethylenoxid.

Anstelle der Verbindungen II kann man auch die entsprechenden Chlorhydrine verwenden, aus denen durch den Einfluß des Amins intermediär das Epoxid entsteht.

Als polyfunktionelle Alkohole eignen sich z.B. Glycerin, Pentaerythrit, Trimethylolpropan und Sorbit. Als Mono- bzw. Disaccharide kommen insbesondere Glucose und Rohrzucker in Betracht.

Geeignete wasserlösliche Amine sind Ammoniak, primäre aliphatische Amine wie Methyl-, Ethyl-, Propyl- oder Butylamin, primäre oder sekundäre aliphatische Diamine, z.B. $H_2N-(CH_2)_n-NH_2$ mit n = 2 bis 6 und aliphatische Polyimine. Das bevorzugte Amin ist Polyethylenimin. Die Menge an Amin wird so gewählt, daß das Verhältnis von Epoxy- zu Aminogruppen 1 : 1,4 bis 1 : 20, bevorzugt 1 : 1,7 bis 1 : 6 beträgt. Um eine Schädigung des zu immobilisierenden Enzyms durch zu stark alkalische Bedingungen zu vermeiden, empfiehlt es sich, das Amin vor der Abmischung partiell mit einer Säure zu neutralisieren. Besonders geeignet hierfür sind einbasige Mineralsäuren wie Salzsäure oder Salpetersäure. Der anzustrebende pH-Wert liegt vorzugsweise zwischen 8 und 9.

Das Verhältnis von Epoxid und Amin zur Menge an enzymatisch aktiver Lösung bzw. Suspension ist so zu wählen, daß einerseits die Löslichkeit der beiden reaktiven Komponenten gewährleistet ist und andererseits kein zu weiches Gel durch eine zu starke Verdünnung des Systems entsteht. Das richtige Verhältnis der beiden Bestandteile läßt sich am besten durch einen Vorversuch ermitteln.

Bezüglich der Reihenfolge der Abmischung ist es vorzuziehen, erst das Epoxid und dann das Amin zuzugeben. Die Temperatur während der Abmischung kann zwischen 0 °C und der Temperatur liegen, bei der eben noch keine erhebliche Denaturierung des zu immobilisierenden Enzyms auftritt. Bevorzugt wird die Abmischung bei Raumtemperatur durchgeführt. Für die Temperatur während der Gelbildung gelten die gleichen Regeln.

Enzyme bzw. emzymhaltiges Material (Zellen und Zellbestandteile) können bis zu solchen Konzentrationen in das Gel eingearbeitet werden, daß entweder die mechanische Festigkeit des Gels darunter leidet oder aber die Aktivität des Enzyms infolge Diffusionslimitierung nicht mehr voll genutzt werden kann. Bei reinem Enzym liegt die Obergrenze in der Größenordnung von 30 bis 50 mg/g Gel.

Elastizität und Dichte der enzymatisch aktiven Gele können variiert werden, wenn der gelbildenden Mischung feinteilige Inertmaterialien wie z.B. Aktivkohle, Kieselgur oder solche Materalien, wie sie zur Verstärkung von Kunststoffen verwendet werden, oder aber Kunststoff-Pulver in einer Menge von bis zu 100 Gew.%, bezogen auf die gelbildende Mischung, beigefügt werden. Es empfiehlt sich, die Verträglichkeit des Inertmaterials für das Enzym in einem kleinen Probeansatz zu ermitteln.

Ein weiterer Weg zur Erzielung eines enzymatisch aktiven Präparates mit besonders hoher Festigkeit ist die Einarbeitung der gelbildenden Mischung in die Poren eines inerten, porösen Materials wie z.B. Kieselgur, Glasfaservlies, poröse Ionenaustauscher, offenzellige Schäume, synthetische Adsorbentien, poröse Mineralien wie Ton und Bimsstein. Umgekehrt ist es auch möglich; z.B. zur Variation der Dichte oder der Kompressibilität, inerte Substanzen wie die soeben genannten oder Sand oder anderes kleinteiliges Material in das Gel einzuarbeiten. Schließlich kann das enzymatisch aktive Gel auch dadurch zusätzlich verfestigt werden, daß es vor seiner Verwendung getrocknet wird.

Das Gel kann auch in wasserfreien bzw. weitestgehend wasserfreien Lösungsmitteln eingesetzt werden, wenn dies durch die durchzuführende Reaktion angezeigt und das immobilisierte Enzym unter diesen Umständen aktiv ist.

Bei der Immobilisierung von Enzymen, welche zur Entfaltung ihrer Aktivität Metallionen benötigen, empfiehlt es sich, der gelbildenden Mischung schwerlösliche Oxide oder Salze des entsprechenden Metalls

- möglichst in feingepulverter Form - zuzusetzen.

Das nach dem vorliegenden Immobilisierungsverfahren entstandene Gel kann zur Vermeidung eines diffusionsbedingten Aktivitätsverlustes in Form eines Films hergestellt oder aber zerkleinert werden. Hierzu wird es beispielsweise durch ein Sieb gedrückt oder durch einen Extruder geschickt.

Den technischen Erfordernissen des Einsatzes entsprechend ist dabei eine Teilchengröße zwischen ca. 0,1 und 5 mm anzustreben. Einen weiteren Weg zur Erzielung eines feinteiligen Gels stellt die Suspendierung der gelbildenden Mischung in einem organischen, nicht mit Wasser mischbaren Lösungsmittel dar. Dabei wird die Suspension so lange gerührt, bis die Gelbildung beendet ist. Man erhält schließlich ein Gel in Form kleiner Perlen mit einem Durchmesser von ca. 0,2 bis 2 mm, das mit Hilfe eines Siebes von dem Lösungsmittel abgetrennt werden kann.

Das vorliegende Immobilisierungsverfahren zeichnet sich durch besondere Einfachheit aus, da es nur die Schritte Abmischen, Aushärten und Zerkleinern umfaßt. Durch die Verwendung wasserlöslicher Epoxide wird eine innige Durchmischung von Epoxid und Enzym erreicht, was eine kovalente Bindung des Enzyms an das entstehende Gel mit Hilfe der Epoxidgruppen erleichtert. Besonders überraschend ist die außerordentlich starke Erhöhung der Lebensdauer von D-Hydantoinase nach der Immobilisierung gemäß dem vorliegenden Verfahren.

Die folgenden Beispiele sollen die Erfindung näher erläutern.

Beispiel 1

Herstellung von Glycidylethern

a)

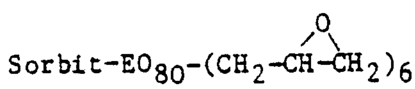

3705 g Sorbit-$EO_{80}$ (Reaktionsprodukt aus 1 Mol Sorbit mit 80 Mol Ethylenoxid, vgl. Houben-Weyl, Methoden der Org. Chemie 14/2, (1963), S. 450) werden mit 14,8 g $BF_3$-dihydrat versetzt. Danach werden bei 70 °C unter Rühren 555 g Epichlorhydrin zugetropft. Es wird weitere 2 Stunden bei 70 °C gerührt und anschließend bei 20 bis 35 °C 528 g 50 %ige Natronlauge im Verlauf von 1 bis 2 Stunden zugetropft. Es wird so lange weitergerührt, bis etwa 90 % der Natronlauge verbraucht sind. Der Natronlauge-Verbrauch wird titrimetrisch verfolgt.

Die Hauptmenge des Wassers wird bei 70 °C im Wasserstrahlvakuum abdestilliert und der Rest heiß (70 °C) abgesaugt.

So werden 3439 g (85 %) Umsetzungsprodukt von Epichlorhydrin mit Sorbit-$EO_{80}$ erhalten, welches einen Epoxid-Titer von 1,2 bis 1,3 mVal/g besitzt. Das Produkt besitzt die Formel II, in der X der Sorbitrest, $R^1$, $R^2$ und $R^3$ Wasserstoffatome, m = 1,34 und n = 6 sind.

Analog a) wurden hergestellt:

b) Umsetzungsprodukt von Epichlorhydrin mit Pentaerythrit-$EO_{40}$, Ausbeute 86 %, Epoxid-Titer: 1,6 mVal/g. Das Produkt besitzt die Formel II, in der X der Pentaerythritrest, $R^1$, $R^2$ und $R^3$ Wasserstoffatome, m = 10 und n = 4 sind.

c) Wie b), aber mit Pentaerythrit-$EO_{57}$, Ausbeute 83 %, Epoxid-Titer: 0,9 mVal/g, m = 14,3, n = 4.

d) Umsetzungsprodukt von Epichlorhydrin mit Glycerin-$EO_{60}$, Ausbeute 78 %, Epoxid-Titer: 0,9 mVal/g. Das Produkt besitzt die Formel II, in der X der Glycerinrest, $R^1$, $R^2$ und $R^3$ Wasserstoffatome, m = 20, n = 3 sind.

e) Umsetzungsprodukt von Epichlorhydrin mit Sorbit-$EO_{30}$, Ausbeute 84 %, Epoxid-Titer 1,9 mVal/g. Das Produkt besitzt die Formel II, in der X den Sorbitrest, $R^1$, $R^2$ und $R^3$ Wasserstoffatome, m = 5, n = 6 sind.

f) Umsetzungsprodukt von Epichlorhydrin mit Trimethylolpropan-$EO_{40}$, Ausbeute 81 %, Epoxid-Titer 1,2 mVal/g. Das Produkt besitzt die Formel II, in der X der Trimethylolpropanrest, $R^1$, $R^2$ und $R^3$ Wasserstoffatome, m = 13,4, n = 3 sind.

Beispiel 2

Immobilisierung von Lipase

Durch Abmischen im Becherglas bei Raumtemperatur in der unten angegebenen Reihenfolge und Stehenlassen über Nacht wurden Gele hergestellt, die durch Drücken durch ein Sieb mit 2 mm Maschenweite zerkleinert wurden:

a) 10 g Lösung von 2,3 g Pankreas-Lipase (Fluka-Nr. 62.000) in 7,7 g 0,05 M Tris(tris-Hydroxymethylamin-Hydrochlorid)-Puffer mit pH 8,5 (M = molar)
6,5 g Glycidylether, hergestellt entsprechend Beispiel 1a)
4 g Polyethyleniminlösung, mit Salzsäure auf pH 9,0 eingestellt.
Molgewicht des Polyethylenimins ca. 40.000. Anteil Polyethylenimin: 25 Gew.%.

b) 5 g Lösung von Pankreas-Lipase (siehe a))
5 ml 0,05 M Tris-Pufferlösung, pH 8,5
6,5 g Glycidylether entsprechend Beispiel 1a
4 g Polyethylenimin-Lösung (siehe a)).

Die Ermittlung der Lipase-Aktivität erfolgte bei 30 °C in einer pH-statisch arbeitenden Apparatur. Es wurde 1 g Tributyrin (Glycerintributyrat) in 50 ml 0,02 M Borax/HCl-Pufferlösung, pH 7,5, emulgiert.

Zum Vergleich wurden unter starkem Rühren 25 mg nicht immobilisierte Pankreas-Lipase eingesetzt. Zur Konstanthaltung des pH-Wertes wurde 0,5 N Natronlauge zudosiert. Es ergab sich ein Verbrauch von 3,3 µmol NaOH/mg Lipase . min.

Die Wiederholung dieses Versuchs mit äquivalenten Mengen Lipase in immobilisierter Form ergabe folgende Werte:

Gel a) 0,29 µmol NaOH/mg Lipase . min
Gel b) 0,26 µmol NaOH/mg Lipase . min.

Damit ist erwiesen, daß die generell schwierig immobilisierbare Lipase nach diesem Verfahren immobilisierbar ist.

Beispiel 3

Immobilisierung von Subtilisin

14 ml einer 0,02 M Borax-Pufferlösung vom pH 7,5, die gleichzeitig 0,002 M an Zinksulfat war und 60 mg Subtilisin (Protease Typ VIII, Fa. Sigma) in den 14 ml enthielt, wurden in einem Becherglas bei Raumtemperatur mit 9,75 g Glycidylether entsprechend Beispiel 1b) sowie mit 6 g Polyethylenimin/HCl-Lösung (Molgewicht des Polyethylenimins ca. 40.000; pH 8,5; Polyethylenimin: 25 Gew.%) gemischt. Die Mischung härtete über Nacht aus und wurde durch Drücken durch ein Sieb mit einer Maschenweite von 0,8 mm zerkleinert.

Die Ermittlung der enzymatischen Aktivität erfolgte durch Spaltung von H-D-Valyl-L-leucyl-L-lysin-p-nitroanilid-dihydrochlorid

(H-D-Val-L-Leu-Lys-NH-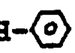-$NO_2$.2 HCl),

wobei die Menge an freigesetztem p-Nitroanilin (ε = 9,62 l/mmol.cm) durch Messung der Lichtabsorption bei 405 nm bestimmt wurde.

Eingesetzte Lösungen:

Puffer: Borax, 0,02 M, pH 7,5.
Substrat: S 2251

(H-D-Val-L-Leu-Lys-NH-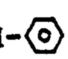-$NO_2$.2 HCl;

Fa. Kasi Diagnostica, Stockholm, Schweden). Das Substrat wurde in Borax-Pufferlösung (s.o.) in einer Konzentration von 5 mg/ml eingesetzt.
Enzym: Protease Typ VIII, Fa. Sigma. Für Vergleichsmessungen wurde das Enzym in Borax-Pufferlösung (s.o.) in einer Konzentration von 1,5 mg/ml eingesetzt.

Die Durchführung der Aktivitätsbestimmung erfolgte derart, daß bei 23 °C 1 ml Substratlösung, 1,6 ml

Pufferlösung und 400 µl Enzymlösung bzw. ein Äquivalent an immobilisiertem Enzym in eine Küvette von 1 cm Dicke gegeben wurden und dann die Küvette 10 min lang langsam und stetig von Hand geschüttelt wurde. Danach wurde die Extinktion gemessen. Bei Verwendung des immobilisierten Enzyms mußte zuvor das Immobilisat durch kurzes Zentrifugieren (10 s) sedimentiert werden. Für das freie Enzym ergab sich eine Aktivität von 0,024 µmol p-Nitroanilin/mg Enzym . min. Der entsprechende Wert für das immobilisierte Enzym betrug 0,019 µmol p-Nitroanilin/mg Enzym . min.

Beispiel 4

Immobilisierung von D-Hydantoinase

Es wurde die 10 gew.%ige, wäßrige, mittels Ultraschall aufgeschlossene Suspension des lyophilisierten, D-Hydantoinase-aktiven Mikroorganismus CBS 303.80 (DE-OS 30 31 151) eingesetzt. Die Herstellung der Immobilisate erfolgte durch Abmischen der unten angeführten Komponenten in einem Becherglas bei Raumtemperatur in der angegebenen Reihenfolge, Aushärtung über Nacht und Zerkleinerung des entstandenen Gels durch Drücken durch ein Sieb der Maschenweite 1 mm.

Einsatzstoffe:

| | |
|---|---|
| Versuch 4a) | 2,5 ml Suspension des Mikroorganismus CBS 303.80 (s.o.) |
| | 1 g Glycidylether, entsprechend Beispiel 1e) |
| | 0,9 ml Methylamin, 100 %ig |
| Versuch 4b) | 2,5 ml Suspension des Mikroorganismus CBS 303.80 (s.o.) |
| | 1 g Glydicylether, entsprechend Beispiel 1e) |
| | 1,2 ml 3-(2-Aminoethyl)aminopropylamin |
| Versuch 4c) | 2,5 ml Suspension des Mikroorganismus CBS 303.80 (s.o.) |
| | 1 g Glycidylether, entsprechend Beispiel 1c) |
| | 0,05 g Braunstein, fein gepulvert |
| | 1 g Polyethylen-Lösung, mit Salzsäure auf pH 9 eingestellt. Das Molgewicht des Polyethylenimins betrug ca. 40.000; Anteil Polyethylenimin 25 Gew.%. |

Die Ermittlung der enzymatischen Aktivität erfolgte bei 60 °C in einer pH-statisch arbeitenden Apparatur. Als Substrat wurde 5-Methylthioethylhydantoin in einer Konzentration von 10 mg/ml in 0,1 M Tris/HCl-Pufferlösung (pH 8,5) verwendet. Die Aktivität wurde über den Verbrauch an 1 n Natronlauge ermittelt. Im Vergleichstest wies der freie, ultraschall-aufgeschlossene Mikroorganismus CBS 303.80 eine Aktivität auf, die zum Verbrauch von 402 µmol NaOH/g Mikroorganismus (trocken) . min führte. Für die Immobilisate ergaben sich folgende Werte:
Versuch 4a): 1 µmol NaOH/g Immobilisat . min
Versuch 4b): 2 µmol NaOH/g Immobilisat . min
Versuch 4c): 11 µmol NaOH/g Immobilisat . min

Nach 1 Woche Einsatz besaßen die Immobilisate noch über 50 % ihrer Anfangsaktivität, während die Aktivität des freien Mikroorganismus nach ca. 4 Stunden zum Erliegen kam.

**Patentansprüche**

**1.** Verfahren zur Immobilisierung von Enzymen oder Mikroorganismen, dadurch gekennzeichnet, daß man folgende Bestandteile in wäßriger Lösung miteinander mischt:

a) ein Enzym oder einen dispergierten Mikroorganismus,
b) einen wasserlöslichen Glycidylether der Formel II

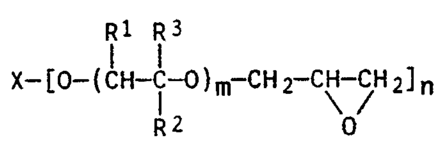

II,

worin m eine Zahl von 1 bis 30, n eine ganze Zahl von 2 bis 10, X der Rest eines polyfunktionellen Alkohols mit 2 bis 6 Kohlenstoffatomen oder eines Mono- oder Disaccharids sind und die Reste $R^1$,

$R^2$ und $R^3$ Wasserstoffatome oder Methyl- oder Ethylgruppen darstellen,

c) ein wasserlösliches Amin mit mindestens zwei N-H-Gruppen, gegebenenfalls in partiell neutralisierter Form,

und die so erhaltene Mischung gelieren läßt.

**2.** Verfahren gemäß Anspruch 1, dadurch gekennzeichnet, daß man als Enzym Lipase, alkalische Protease oder D-Hydantoinase verwendet.

## Claims

**1.** A process for immobilizing an enzyme or microorganism, wherein the following components are mixed with one another in aqueous solution:

a) an enzyme or a dispersed microorganism,

b) a water-soluble glycidyl ether of the formula II

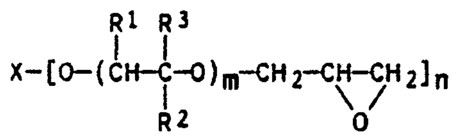

II

where m is from 1 to 30, n is an integer from 2 to 10, X is the radical of a polyfunctional alcohol of from 2 to 6 carbon atoms or of a mono- or disaccharide and $R^1$, $R^2$ and $R^3$ are each hydrogen or methyl or ethyl, and

c) a water-soluble amine possessing two or more NH groups,

if necessary in a partially neutralized form,

and the mixture thus obtained is allowed to gel.

**2.** A process as claimed in claim 1, wherein the enzyme used is lipase, alkaline protease or D-hydantoinase.

## Revendications

**1.** Procédé d'immobilisation d'enzymes ou de microorganismes caractérisé par le fait qu'on mélange ensemble, en solution aqueuse, les composants suivants :

a) un enzyme ou microorganisme dispersé

b) un éther glycidylique soluble dans l'eau de la formule II

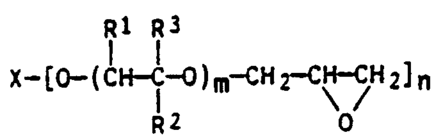

(II)

où m est un nombre de 1 à 30, n un nombre entier de 2 à 10, X le reste d'un alcool polyfonctionnel ayant 2 à 6 atomes de carbone, ou un mono-ou disaccharide et les restes $R^1$, $R^2$ et $R^3$ représentent des atomes d'hydrogène ou des groupes méthyle ou éthyle

c) une amine soluble dans l'eau ayant au moins deux groupes N-H, éventuellement sous forme partiellement neutralisée

et on fait gélifier le mélange ainsi obtenu.

**2.** Procédé selon la revendication 1, caractérisé par le fait que l'on utilise, comme enzyme, la lipase, la protease alcaline ou la D-hydantoinase.